# EUROPÄISCHE PATENTSCHRIFT

(11) **EP 1 023 164 B1**
(45) Veröffentlichungstag und Bekanntmachung des Hinweises auf die Patenterteilung: **07.04.2004**
(21) Anmeldenummer: 98951458.3
(22) Anmeldetag: 23.09.1998
(51) Int. Cl.: B32B 5/26, D04H 13/00

(54) **VERBUNDMATERIAL UND DESSEN VERWENDUNG SOWIE VERFAHREN ZU SEINER HERSTELLUNG**
COMPOSITE, ITS USE, AND METHOD FOR ITS PRODUCTION
MATERIAU COMPOSITE, SON UTILISATION ET SON PROCEDE DE PRODUCTION

(30) Priorität: 07.10.1997 DE 19744231
(43) Veröffentlichungstag der Anmeldung: 02.08.2000
(73) Patentinhaber: Paul Hartmann Aktiengesellschaft, 89522 Heidenheim (DE)
(72) Erfinder: MALOWANIEC, Krzysztof, Daniel, D-89522 Heidenheim (DE); OLTMANN, Eckhard, D-89520 Heidenheim (DE); DENK, Bettina, D-89231 Neu-Ulm (DE)
(74) Vertreter: Friz, Oliver
(86) Internationale Anmeldenummer: PCT/EP1998/006067
(87) Internationale Veröffentlichungsnummer: WO 1999/017927

(56) Entgegenhaltungen:
- EP-A- 0 775 572

## Beschreibung

Die Erfindung betrifft ein Verbundmaterial zur Bildung einer flüssigkeitsrückhaltenden Schicht bei einem Hygieneartikel oder einem medizinischen Produkt, mit einer ersten Schicht im wesentlichen endloser Spinnfasern mit einem Durchmesser von 15-35 µm und einer zweiten Folienschicht.

Verbundmaterialien aus Vlies- und Folienkomponenten sind bekannt, z.B. aus dem Bereich der zum einmaligen Gebrauch bestimmten Hygieneartikel.

Als Rückenblattmaterial dieser Hygieneartikel wurde früher ausschließlich auf Kunststoffolien zurückgegriffen, was dem Hygieneartikel allerdings einen zunehmend nicht mehr akzeptierten plastik-ähnlichen Eindruck vermittelt. So finden heute vermehrt zweischichtige Vlies-Folien-Laminate als Rückenblatt dieser Hygieneartikel Verwendung, wie zum Beispiel in der EP 0 187 728 B1 offenbart: Die innen zu liegen kommende Folienkomponente übernimmt dabei im wesentlichen die Abdichtfunktion, während die außen zu liegen kommende Vlieskomponente dem Rückenblatt einen faserigen, textil-ähnlichen Eindruck vermitteln soll. Als Vlieskomponente werden bevorzugt Spinn- oder Kardenvliese eingesetzt, die auf Basis relativ groben Fasermaterials (Durchmesser >15 µm) hergestellt werden.

Aus der DE 44 29 251 C2 ist eine zumindest zweischichtige Laminatkonstruktion bestehend aus einer textilen Trägerschicht und einer weiteren Folienschicht bekannt. Eine Spinnfaserschicht ist nicht vorgesehen.

Die DE 41 08 937 A1 offenbart ein Verbundvliesmaterial, welches aus einer Mischung von feinen Mikrofasern und demgegenüber groben Filamenten gebildet ist, sowie die Verwendung dieses Verbundvliesmaterials als körperzugewandte Abdeckschicht bei einem Hygieneartikel.

Aus der WO 97/16148 ist eine flüssigkeitsrückhaltende Schicht aus einem drei- oder vierlagigen Faserverbundmaterial bekannt, wobei eine Spinnvliesschicht und zwei Meltblown-Schichten oder in abwechselnder Reihenfolge Spinnvliesschichten und Meltblown-Schichten eingesetzt werden. Eine Folienschicht ist nicht vorgesehen.

Aus der WO 96/07376 ist ein Hygieneartikel mit einer flüssigkeitsrückhaltenden Schicht bestehend aus einer Folienschicht oder einer Mikrofaserschicht und einer damit verbundenen strukturbildenden Faserschicht mit einer Schmelzkleberkomponente bekannt.

Es hat sich nun allerdings gezeigt, daß diese faserige, textil-ähnliche Gestaltung des Rückenblattes eines Hygieneartikels zum einmaligen Gebrauch neben den optischen und taktilen Vorteilen mit erheblichen Nachteilen, subjektiv empfundenen aber auch objektiven, behaftet ist. So empfinden die Verwender dieser Hygieneartikel, die bisher eine einfache. aber glatte Folie als Rückenblatt gewohnt waren, das faserige Rückenblattmaterial oftmals als zu rauh.

Des weiteren besteht die Gefahr, während der Manipulation dieses Hygieneartikels, z.B. beim Wickeln eines Babys, mit Schmuckgegenständen wie Ringen oder auch Uhren an der faserigen Oberflächenstruktur des Rückenblattmaterials zu verhaken, wobei dieses sogar zerstört werden kann.

Bei Verwendung des bekannten Vlies-Folien Laminates als Rückenblattmaterial einer Wegwerfwindel und gleichzeitiger Verwendung der inzwischen weit verbreiteten Klettverschlußelemente greifen die Hakenelemente der Verschlußstreifen nicht nur in den dafür vorgesehenen Zielbereich, der üblicherweise ein im Bauchbereich auf der Außenseite des Rückenblattes positioniertes Flauschmaterial ist, ein, sondern verhaken außerdem in unerwünschter Weise überall auf der faserigen Oberfläche des Rückenblattmaterials.

Aus der EP-A-0 775 572 ist ein wenigstens zweischichtiges Verbundmaterial bekannt mit einer Spinnfaserschicht und einer Mikrofaserschicht aus Fasern mit einem Durchmesser von weniger als 10 µm; die Schichten werden separat voneinander hergestelt und verfestigt und anschließend zur Bildung eines Laminats miteinander verbunden.

Es ist eine Aufgabe der Erfindung ein verbessertes Verbundmaterial zu schaffen, das insbesondere unter dem Aspekt der Verwendung als Rückenblattmaterial in Hygieneartikeln zum einmaligen Gebrauch die bekannten Nachteile ausschließt.

Diese Aufgabe wird durch ein Verbundmaterial mit den Merkmalen des Anspruches 1 gelöst.

Die mittlere, sandwichartig eingeschlossene Schicht besteht aus im wesentlichen endlosen, thermoplastischen, relativ unorientiert im Spinnverfahren abgelegten Fasern bzw. Filamenten mit einem Durchmesser von 15-35 µm. Das Spinnverfahren zur Herstellung von Spinnvliesstoffen ist dem Fachmann seit langem bekannt und bedarf hier deshalb keiner besonderen Erläuterung. Für die Erzeugung der Spinnfaserschicht können z.B. Polymere aus der Gruppe der Polyolefine, der Polyamide, der Polyester, der Polyurethane und auch entsprechende Copolymere verwendet werden.

Eine äußere Schicht ist aus langen Mikrofasern mit einem Durchmesser < 10 µm hergestellt nach dem ebenfalls dem Fachmann bekannten Meltblown-Verfahren. Bei Verwendung des Verbundmaterials als Rückenblatt eines Hygieneartikels würde diese Schicht bevorzugt außen zu liegen kommen. Für die Erzeugung der Mikrofaserschicht können ebenfalls z.B. Polymere aus der Gruppe der Polyolefine, der Polyamide, der Polyester, der Polyurethane und auch entsprechende Copolymere verwendet werden.

Die Mikrofasern werden nach der Erfindung direkt auf die Spinnfaserschicht im Meltblown-Verfahren aufgebracht. In der Folge greifen die Mikrofasern in die Oberflächenstruktur der Spinnfaserschicht ein und bilden einen Überzug der im Einzelnen betrachtet dreidimensional strukturierten Oberfläche der Spinnfaserschicht.

Die Mikrofaserschicht weist im bevorzugten Fall lediglich ein Flächengewicht von ca. 5 g/m² auf und folgt im Querschnitt betrachtet der dreidimensionalen, faserigen Oberflächenstruktur der mittleren Spinnfaserschicht, das heißt sie greift räumlich in die Struktur dieser Schicht ein.

Eine Spinnfaser/Mikrofaser-Schichtstruktur ist bereits aus der DE-0S 23 56 720 bekannt. Herstellbedingt greift die Mikrofaserschicht aber nicht in eine dreidimensionale Spinnfaserschicht ein. Statt dessen ist ein sehr diskreter, in der Ebene des Laminates homogener Phasenübergang erkennbar.

Die EP 0 403 840 B1 zeigt ebenfalls eine Spinnfaser/Mikrofaser-Struktur, die aber durch ein gleichmäßiges Gemisch der beiden Faserarten gekennzeichnet ist, so daß keine Phasengrenzen mehr erkennbar sind, sondern eine vollständige Durchmischung der Komponenten über den gesamtem Querschnitt gegeben ist.

Im Gegensatz dazu liegt bei der vorliegenden Erfindung keine über den Querschnitt homogene Mischung der beiden Fasermaterialien vor. Da die Mikrofasern makroskopisch betrachtet eine folienähnliche Struktur bilden und diese sich ähnlich einer sehr eng anliegenden Haut über die faserige Oberfläche der Spinnfaserschicht legt, wird sicher vermieden, daß es zu einer ungewollten Verhakung und Verhedderung der Spinnfasern mit scharfkantigen Elementen, z.B., Haken eines Klettverschlußsystems kommt.

Es hat sich gezeigt, daß die Gefahr des ungewollten Verhakens mit scharfkantigen Elementen dann deutlich reduziert ist, wenn die Haken-Peel-Off-Kraft gegenüber der die Außenseite des Verbundmaterials bildenden Mikrofaserschicht kleiner als 20 cN/25mm, bevorzugt kleiner als 10 cN/25mm, besonders bevorzugt kleiner als 5 cN/25mm ist. Die vorstehend als Haken-Peel-Off-Kräfte bezeichneten Haltekräfte werden in der nachstehenden Weise definiert und gemessen. Zum Test wird ein 25 mm breiter Teststreifen eines Hakenmaterials verwendet, das unter der Herstellerbezeichnung CS 200-900 ppi, xMH-4123 von der Fa. Minnesota Mining Manufacturing in Neuss, Deutschland erhalten werden kann. Dieser Teststreifen wird unter einem Anpreßdruck von 2 kg unter Verwendung eines Überrollgeräts auf die zu testende Gegenfläche, also die Oberfläche der Mikrofaserschicht, aufgebracht. Hierfür ist das Verbundmaterial auf einem starren Halter fixiert. Der Halter wird an einem Zugprüfgerät fixiert, und der Teststreifen wird an eine Zugbacke geklemmt, so daß sich einen Abzugswinkel von 150° ergibt, der sich beim Abziehen geringfügig um einige wenige Grad verringert. Unter Messung der als Peel-Off-Kraft bezeichneten Haltekraft wird der Teststreifen mit konstanter Geschwindigkeit von der Gegenfläche abgezogen. Die gemessene Peel-Off-Kraft wird als Funktion des Wegs aufgezeichnet.

Mit der Erfindung wird auch die als unangenehm empfundene Rauhigkeit der Spinnfaserschicht herabgesetzt. Gleichwohl zeichnet sich die faserige Struktur der Spinnfasern auch durch die sehr dünne Mikrofaserschicht hindurch ab, weshalb von dieser Außenseite des Materialverbundes nach wie vor ein optisch und taktil erlebter textil-ähnlicher Eindruck vermittelt wird.

Das Eingreifen der Mikrofaserschicht in die Spinnfaserschicht: hat zudem den Vorteil, daß bei gegebenem Flächengewicht des Verbundmaterials ein höheres Festigkeitsniveau erreicht wird.

Zur besseren Verbindung der Faserschichten kann der Verbund vorteilhafterweise durch eine Vielzahl punktförmiger, durch Kombination von Druck und Temperatur erzeugter Verbindungen in an sich bekannter Weise verfestigt werden. Bevorzugt besitzt eine dieser punktförmigen Verbindungen eine flächenhafte Ausdehnung von nicht mehr als 0,5 mm². Die Anzahl dieser Verbindungspunkte sollte nicht größer als 45000/m² sein, damit das Verbundmaterial seine Drapierfähigkeit erhält.

Die andere der äußeren Schichten wird durch die Kunststoffolie gebildet, die im wesentlichen die Abdichtfunktion übernimmt im Falle, daß das Verbundmaterial als Rückenblatt in Hygieneartikeln Verwendung findet. Die Kunststoffolie ist bevorzugt ebenfalls aus einem thermoplastischen Polymer der Gruppe der Polyolefine, Polyester, Polyamide, Polyurethane oder entsprechender Copolymere hergestellt.

In einer bevorzugten Ausführungsform der Erfindung greift die Kunststoffolie ebenfalls in die faserige, dreidimensionale Struktur der Spinnfaserschicht ein. Dies hat den Vorteil, daß bei gegebenem Flächengewicht ein höheres Festigkeitsniveau des Verbundmaterials erreicht wird.

Durch das räumliche Eingreifen zumindest der Mikrofaserschicht in die faserige Struktur der Spinnvliesschicht resultiert ein mittlerer Abstand zwischen den äußeren Schichten, der kleiner ist als die Dicke der Spinnfaserschicht, wenn diese als die im Verbundmaterial größte Entfernung senkrecht zur flächenhaften Erstreckung des Verbundmaterials zwischen den Oberflächen der Spinnfaserschicht definiert ist.

Präparationstechniken wie das Herstellen geeigneter Schnitte, ggf. Mikrotomschnitte, ggf. nach Einbetten des Verbundmaterials in ein dem Verbundmaterial eine hohe Integrität verleihendes Polymer und mikroskopisch unterstützte Analysemethoden zur Bestimmung der oben genannten Meßgrößen sind dem Fachmann bekannt, so daß dies an dieser Stelle keiner näheren Erläuterung bedarf.

Insbesondere im Hinblick auf die Verwendung des Materials als Rückenblatt in Hygieneartikeln ist in einer weiteren bevorzugten Ausführungsform der Erfindung das Verbundmaterial atmungsaktiv ausgeführt. Das heißt es besitzt eine Atmungsaktivität von mindestens 500 g/m² über einen Zeitraum von 24h, ermittelt nach DIN 53122 Blatt 1. Gleichzeitig sollte das Verbundmaterial bei Verwendung als Rückenblatt in Hygieneartikeln unter den dort herrschenden Tragebedingungen flüssigkeitsdicht sein, d.h. kein Wasser in flüssiger Form passieren lassen. Unter flüssigkeitsdicht in diesem Sinne wird eine Wassersäule von mindestens 250 mm, ermittelt nach DIN EN 20811 verstanden.

Der Verbund der beiden Faserschichten kann per se als atmungsaktiv angesehen werden. Somit ist als Folienkomponente des Verbundmaterials ein atmungsaktives Folienmaterial auszuwählen. Diese Materialien sind dem Fachmann bekannt (z.B. DE-PS 31 21 040, DE-0S 33 06 843; G. Pinchard (presentation "Breathable Films" at "Absorbent Products Conference", Oct. 17, 1996 in San Antonio, Texas, USA). Grundsätzlich besteht die Möglichkeit, eine mit Mikroporen versehene Folie einzusetzen, um Wasserdampf die Möglichkeit zu geben, auf mechanischem Wege zu penetrieren oder Folien zu verwenden, die Wasserdampf mithilfe der Chemisorption penetrieren lassen, wie es beispielsweise bei Zellglasfolien seit langem bekannt ist. Bei Verwendung einer mikroporösen Folie weisen die Poren - bei hinsichtlich ihrer Geometrie idealisiert runder Betrachtung der Poren - bevorzugt einen durchschnittlichen Durchmesser von 0,2-10 µm auf.

Gemäß einer weiteren bevorzugten Ausführungsform weist das Verbundmaterial und zumindest abschnittsweise Makroporen auf. Unter Makroporen werden alle Art von Öffnungen verstanden, unabhängig von deren Geometrie und unabhängig von der Art und dem Zeitpunkt des Einbringens der Öffnungen. Im Falle der Verwendung des Materialverbundes als Rückenblatt in Hygieneartikeln gewährleisten die Makroporen einen Luftaustausch zwischen der Haut des Trägers und der Außenseite des Hygieneproduktes. Die einzelnen Makroporen weisen bevorzugt eine Projektionsfläche von mindestens 0,1 mm² höchstens aber 5,0 mm², wobei der Anteil der offenen Fläche nicht größer als 25% sein sollte.

Das Vorhandensein von Makroporen kann auf die Folienkomponente des Materialverbundes beschränkt sein. Dies insbesondere dann, wenn der Mikrofaser/Spinnfaserverbund bereits eine ausreichend hohe Luftdurchlässigkeit besitzt. Die Makroporen können aber auch in dem Mikrofaser/Spinnfaserverbund ausgebildet sein, bevorzugt handelt es sich dann um Poren, die in Form sich durch das gesamte Verbundmaterial hindurcherstreckende Öffnungen vorliegen.

Es liegt desweiteren die Aufgabe zugrunde, ein Verfahren zum Herstellen eines erfindungsgemäßen Verbundmaterials anzugeben. Diese weitere Aufgabe wird durch ein Verfahren mit den Merkmalen des Anspruchs 20 gelöst.

Weitere Merkmale, Einzelheiten und Vorteile der Erfindung ergeben sich aus den beigefügten Patentansprüchen und der zeichnerischen Darstellung und nachfolgenden Beschreibung einer bevorzugten Ausführungsform des erfindungsgemäßen Verbundmaterials und eines Verfahrens zu seiner Herstellung. In der Zeichnung zeigt:
- Figur 1: eine Schnittansicht senkrecht zur Ebene eines erfindungsgemäßen Verbundmaterials;
- Figur 2: einen Schnitt nach Figur 1 in vergrößerter Darstellung;
- Figur 3: eine Anordnung zum Herstellen eines Verbunds einer Spinnfaserschicht und einer Mikrofaserschicht;
- Figur 4: eine Anordnung zum Aufbringen einer Folienschicht auf den nach Figur 3 hergestellten Verbund; und
- Figur 5: eine zweite Ausführungsform einer Anordnung zum Aufbringen einer Folienschicht auf den nach Figur 3 hergestellten Verbund.

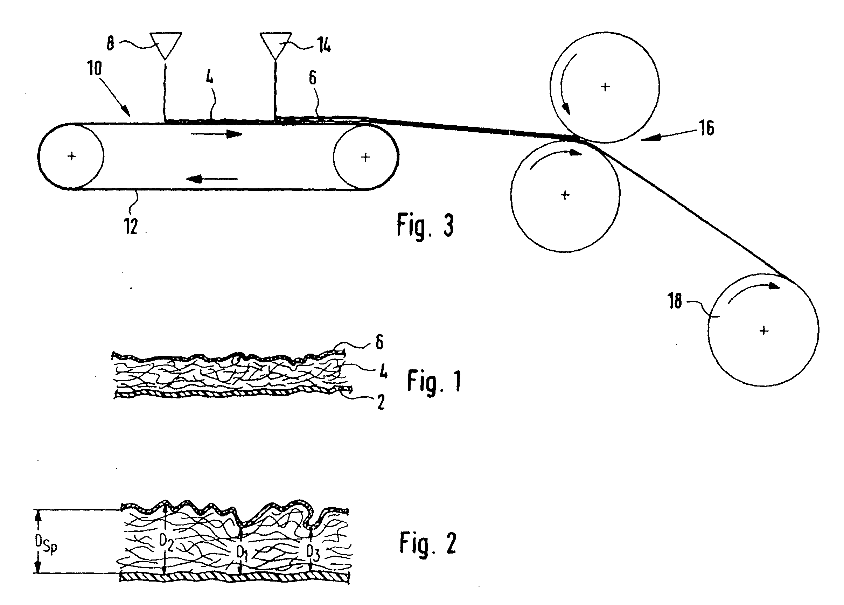

Figur 1 zeigt ein Verbundmaterial, bestehend aus einer eine Außenseite bildenden Folienschicht 2, einer innenliegenden Spinnfaserschicht 4 und einer auf die Spinnfaserschicht 4 im Melt-Blown-Verfahren aufgebrachten Mikrofaserschicht 6.

Wie aus Figur 1 und aus der vergrößerten Darstellung nach Figur 2 ersichtlich, greift die Mikrofaserschicht 6 in die Oberflächenstruktur der Spinnfaserschicht 4 räumlich ein und bildet einen diese Oberflächenstruktur abdeckenden Überzug, der in Folge der Beschaffenheit der Mikrofaserschicht eine gewiße Glättung der Oberfläche der Spinnfaserschicht 4 bewirkt.

Wenn der Abstand Dᵢ der nach innen gewandten Seite der Mikrofaserschicht 6 von der ebenfalls nach innen gewandten Seite der Folienschicht 2 an verschiedenen Stellen i bestimmt und nach (D₁ + D₂ + ... + Dᵢ)/ᵢ = D''bestimmt wird, so ist dieser mittlere Abstand D' geringer als die Dicke D_{Sp} der Spinnfaserschicht, wenn diese als größte Entfernung zwischen zwei Punkten der nach außen weisenden Oberfläche der Spinnfaserschicht senkrecht zur Ebene des Verbundmaterials definiert wird.

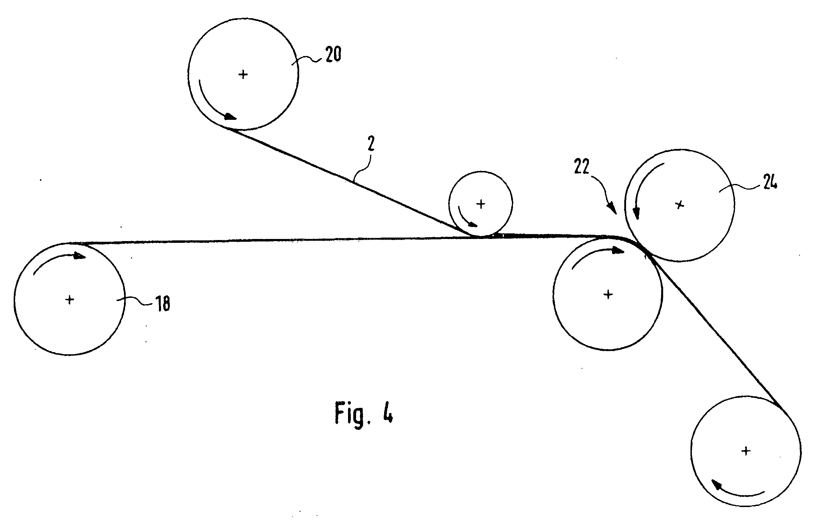

In den Figuren 3 und 4 ist die Herstellung des erfindungsgemäßen Verbundmaterials erläutert.

Es wird zunächst in bekannter Weise eine Spinnfaserschicht 4 gebildet. Durch eine Spinneinheit 8 erfolgt das Schmelzen eines thermoplastischen Polymers, das Ausstoßen des geschmolzenen Polymers durch geeignete Spinndüsen, das Verstrecken der Filamente durch z.B. einen Luftstrom, das Abkühlen und das Zuführen der Fasern auf ein Ablagesystem 10, bevorzugt ein sich kontinuierlich in einer Richtung fortbewegendes Endlossiebband 12. Im bevorzugten Fall werden die Filamente vor dem Ablegen auf das Siebband 12 soweit heruntergekühlt, daß im wesentlichen keine thermischen Schmelzbindungen an den nach dem Ablegen auf das Siebband vorhandenen Kreuzungspunkten der Filamente auftreten. Auf diese noch unkonsolidierte, noch nicht verdichtete und damit offene, eine dreidimensionale Oberflächenstruktur aufweisende Spinnfaserschicht wird bevorzugt in einer integrierten Fertigungslinie durch eine Meltblown-Einheit 14 die Mikrofaserschicht 6 nach dem bekannten Meltblown-Verfahren aufgebracht. Durch Hochgeschwindigkeitsheißluftströme werden die aus der Polymerschmelze direkt unterhalb der Spinndüsen austretenden Filamente auf einen sehr kleinen Durchmesser (< 10 µm) verstreckt und vielfach auch zerrissen, so daß mehr oder weniger lange im Verhältnis zum Durchmesser aber praktisch endlose Mikrofasern gebildet werden. Diese werden kontinuierlich direkt auf die offene Spinnfaserschicht 4 abgelegt, so daß die Mikrofasern in die Oberflächenstruktur der Spinnfaserschicht räumlich eingreifen können.
Anschließend werden die gebildeten Faserschichten 4, 6 durch eine Kalandereinrichtung 16, das heißt durch Anwendung von Druck und Temperatur, verdichtet und verfestigt und auf eine Mutterrolle 18 aufgewickelt. Besitzt die Kalandereinrichtung 16 eine Prägewalze so werden die oben erwähnten besonders verdichteten, punktförmigen Bereiche gebildet.

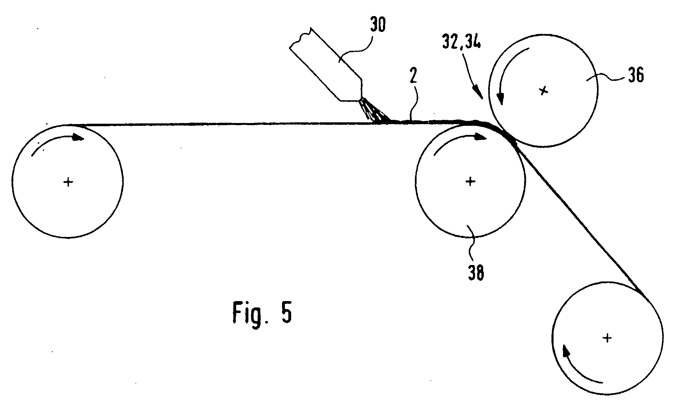

In einem zweiten Verfahrensschritt wird der so gebildete Mikrofaser/Spinnfaserverbund spinnfaserseitig entweder durch eine vorgefertigte Folie kaschiert (Figur 4) oder die Folie wird direkt aus einer Polymerschmelze auf den vorgefertigten Faserverbund extrudiert (Figur 5).

Im ersten Fall werden der Mikrofaser/Spinnfaserverbund und die vorgefertigte Folie 2 kontinuierlich von einer Mutterrolle 18 bzw. 20 abgerollt und einer Kalandereinheit 22 zugeführt. Zumindest eine der Kalanderwalzen 24 ist derart beheizt, daß zumindest die Folie 2 im Preßspalt der Kalandereinheit 22 zumindest abschnittsweise auf eine Temperatur oberhalb ihres Erweichungspunktes/Schmelzpunktes gebracht wird. Auf diese Weise kommt es zu Schmelzverbindungen zwischen Folie 2 und Mikrofaser/Spinnfaserverbund, wobei die Folie 2 in die Oberflächenstruktur der Spinnfaserschicht 4 räumlich eingreifen kann.

Im zweiten Fall nach Figur 5 wird die Folie 2 wie bereits erwähnt direkt aus der Polymerschmelze durch einen Extruder 30 auf die sich kontinuierlich unter dem Extruder fortbewegende Mikrofaser/Spinnfaserschicht extrudiert. Das im Moment des Aufbringens auf den Faserverbund noch geschmolzene und damit viskose Folienmaterial dringt in diesem bevorzugten Fall, unterstützt durch eine sich an die Extrusion anschließende Verfestigungsstation 32 in die dreidimensionale Oberflächenstruktur der Spinnvliesschicht 4 ein. Die Verfestigungsstation 32 besteht vorteilhafterweise aus einem Walzenpaar 34. Die auf die Folienoberfläche gerichtete Walze 36 ist vorteilhafterweise eine Antihaft-Walze, z.B. eine mit Silikon beschichtete Walze, während die auf die Vliesoberfläche gerichtete Walze 38 als Kühlwalze ausgestaltet ist.

## Patentansprüche

1. Verbundmaterial als flüssigkeitsrückhaltende Schicht bei einem Hygieneartikel oder einem medizinischen Produkt, mit einer ersten Schicht im wesentlichen endloser Spinnfasern mit einem Durchmesser von 15-35 µm, mit einer zweiten Folienschicht und mit einer dritten zur Bildung eines dreischichtigen Verbundmaterials auf der der Folienschicht (2) abgewandten Seite der Spinnfaserschicht (4) vollflächig vorgesehenen Schicht (6) aus Mikrofasern mit einem Durchmesser von weniger als 10 µm, wobei die Mikrofasern dieser dritten Mikrofaserschicht (6) derart in die Oberflächenstruktur der Spinnfaserschicht (4) räumlich eingreifen, daß der mittlere Abstand (D') zwischen Mikrofaserschicht (6) und Folienschicht (2) geringer ist als die Dicke D_{Sp} der sandwichartig eingeschlossenen Spinnfaserschicht (4), hergestellt, indem die Mikrofasern direkt im Meltblown-Verfahren auf die Oberflächenstruktur der Spinnfaserschicht (4) aufgebracht sind.

2. Verbundmaterial nach Anspruch 1, **dadurch gekennzeichnet, daß** die Halte- oder Haftkraft eines Hakenmaterials gegenüber der durch die Mikrofaserschicht (6) gebildeten Außenseite des Verbundmaterials kleiner als 20 cN/25mm, bevorzugt kleiner als 10 cN/25mm, besonders bevorzugt kleiner als 5 cN/25mm ist.

3. Verbundmaterial nach Anspruch 1 oder 2, **dadurch gekennzeichnet, daß** auch die Folienschicht (2) in die dreidimensionale Oberflächenstruktur der Spinnfaserschicht (4) eingreift.

4. Verbundmaterial nach einem der vorgenannten Ansprüche, **dadurch gekennzeichnet, daß** das Flächengewicht des Verbundmaterials 20-45 g/m², bevorzugt 25-40 g/m² beträgt.

5. Verbundmaterial nach einem der vorgenannten Ansprüche, **dadurch gekennzeichnet, daß** das Flächengewicht des Verbundmaterials 30-35 g/m² beträgt.

6. Verbundmaterial nach einem der vorgenannten Ansprüche, **dadurch gekennzeichnet, daß** das Flächengewicht der Mikrofaserschicht (6) 3-10 g/m² bevorzugt 4-6 g/m² beträgt.

7. Verbundmaterial nach einem der vorgenannten Ansprüche, **dadurch gekennzeichnet, daß** das Flächengewicht der Spinnfaserschicht (4) 15-25 g/m², bevorzugt 18-22 g/m² beträgt.

8. Verbundmaterial nach einem der vorgenannten Ansprüche, **dadurch gekennzeichnet, daß** die Dicke der Folienschicht (2) 9-20 µm, bevorzugt 12-17 µm beträgt.

9. Verbundmaterial nach einem der vorgenannten Ansprüche, **dadurch gekennzeichnet, daß** die Reißfestigkeit des Verbundmaterials mindestens 15 N/25mm, bevorzugt mindestens 18 N/25mm beträgt.

10. Verbundmaterial nach einem der vorgenannten Ansprüche, **dadurch gekennzeichnet, daß** die Folienschicht (2) atmungsaktiv aber flüssigkeitsdicht ist, so daß das Verbundmaterial ebenfalls atmungsaktiv aber flüssigkeitsdicht ist.

11. Verbundmaterial nach Anspruch 10, **dadurch gekennzeichnet, daß** die Folie (2) Wasserdampf durch den Vorgang der Chemisorption durchläßt.

12. Verbundmaterial nach Anspruch 10, **dadurch gekennzeichnet, daß** die Folie (2) Mikroporen zum Durchlassen von Wasserdampf aufweist.

13. Verbundmaterial nach Anspruch 12, **dadurch gekennzeichnet, daß** die Mikroporen einen Durchmesser von 0,2-10 µm aufweisen.

14. Verbundmaterial nach eine der vorgenannten Ansprüche, **dadurch gekennzeichnet, daß** zumindest die Folienschicht (2) zumindest abschnittsweise Makroporen aufweist.

15. Verbundmaterial nach Anspruch 14, **dadurch gekennzeichnet, daß** der Spinnfaser/Mikrofaserverbund ebenfalls Makroporen aufweist, derart daß Makroporen des Spinnfaser/Mikrofaserverbundes und Makroporen der Folienschicht (2) sich durch das Verbundmaterial hindurcherstreckende Öffnungen bilden.

16. Verwendung eines Verbundmaterials nach einem oder mehreren der vorstehenden Ansprüche als flüssigkeitsrückhaltende Schicht bei einem Hygieneartikel zum einmaligen Gebrauch.

17. Verwendung nach Anspruch 16, **dadurch gekennzeichnet, daß** der Hygieneartikel eine Windel, eine Training-Pant, eine Damenbinde, eine Slipeinlage oder eine Inkontinenzvorlage ist.

18. Verwendung nach Anspruch 16 oder 17, **dadurch gekennzeichnet, daß** das Verbundmaterial als Rückenblatt verwendet wird.

19. Verwendung nach Anspruch 18, **dadurch gekennzeichnet, daß** die Mikrofaserschicht (6) auf der Außenseite des Rückenblattes angeordnet wird.

20. Verfahren zur Herstellung des Verbundmaterials nach einem der Ansprüche 1-15 **gekennzeichnet durch** folgende Verfahrensschritte
- Bilden einer Spinnfaserschicht (4) mit einer offenen Oberflächenstruktur
- Aufbringen von Mikrofasern (6) auf die Spinnfaserschicht (4)
- Verfestigung der gebildeten Mikrofaser/Spinnfaserschicht **durch** Einwirken von Druck und einer Temperatur, die oberhalb des Erweichungspunktes der Mikrofasern und/oder der Spinnfasern liegt
- Aufbringen einer vorgefertigten Folie (2) spinnfaserseitig auf den so vorgefertigten Mikrofaser/Spinnfaserverbund
- Verfestigung des Mikrofaser/Spinnfaserverbundes mit der Folie **durch** Einwirken von Druck und einer Temperatur, die oberhalb des Erweichungspunktes zumindest der Folie liegt.

21. Verfahren zur Herstellung des Verbundmaterials nach einem der Ansprüche 1-15 **gekennzeichnet durch** folgende Verfahrensschritte:
- Bilden einer Spinnfaserschicht (2) mit einer offenen Oberflächenstruktur
- Aufbringen von Mikrofasern (6) auf die Spinnfaserschicht
- Verfestigung der gebildeten Mikrofaser/Spinnfaserschicht **durch** Einwirken von Druck und einer Temperatur, die oberhalb des Erweichungspunktes der Mikrofasern und/oder der Spinnfasern liegt
- direktes Extrudieren einer Folie (2) spinnfaserseitig auf den so gebildeten Mikrofaser/Spinnfaserverbund
- Verfestigung des Mikrofaser/Spinnfaser-Folienverbundes

## Revendications

1. Matériau composite en tant que couche retenant les liquides pour un article d'hygiène ou un produit médical, comprenant une première couche de fibres textiles essentiellement continues d'un diamètre de 15 à 35 µm, une deuxième couche de feuille et une troisième couche (6) prévue sur toute la surface de la face de la couche de fibres textiles (4) opposée à la couche de feuille (2) pour former un matériau composite à trois couches et constituée de microfibres d'un diamètre inférieur à 10 µm, les microfibres de cette troisième couche de microfibres (6) s'enchevêtrant dans l'espace dans la structure superficielle de la couche de fibres textiles (4) de telle sorte que la distance moyenne (D') entre couche de microfibres (6) et couche de feuille (2) est inférieure à l'épaisseur D_{Sp} de la couche de fibres textiles (4) prise en sandwich, fabriquée en déposant les microfibres directement suivant un procédé d'extrusion soufflage sur la structure superficielle de la couche de fibres textiles (4).

2. Matériau composite selon la revendication 1, **caractérisé en ce que** la force de retenue ou d'adhérence d'un matériau accrochant par rapport à la face extérieure du matériau composite formée par la couche de microfibres (6) est inférieure à 20 cN/25 mm, de préférence inférieure à 10 cN/25 mm, en particulier de préférence inférieure à 5 cN/25 mm.

3. Matériau composite selon la revendication 1 ou 2, **caractérisé en ce que** la couche de film (2) s'enchevêtre également dans la structure superficielle en trois dimensions de la couche de fibres textiles (4).

4. Matériau composite selon l'une quelconque des revendications précédentes, **caractérisé en ce que** le poids au mètre carré du matériau composite est compris entre 20 et 45 g/m², de préférence entre 25 et 40 g/m².

5. Matériau composite selon l'une quelconque des revendications précédentes, **caractérisé en ce que** le poids au mètre carré du matériau composite est compris entre 30 et 35 g/m².

6. Matériau composite selon l'une quelconque des revendications précédentes, **caractérisé en ce que** le poids au mètre carré de la couche de microfibres (6) est compris entre 3 et 10 g/m², de préférence entre 4 et 6 g/m².

7. Matériau composite selon l'une quelconque des revendications précédentes, **caractérisé en ce que** le poids au mètre carré de la couche de fibres textiles (4) est compris entre 15 et 25 g/m², de préférence entre 18 et 22 g/m².

8. Matériau composite selon l'une quelconque des revendications précédentes, **caractérisé en ce que** l'épaisseur de la couche de feuille (2) est comprise entre 9 et 20 µm, de préférence entre 12 et 17 µm.

9. Matériau composite selon l'une quelconque des revendications précédentes, **caractérisé en ce que** la résistance à la déchirure du matériau composite est d'au moins 15 N/25 mm, de préférence d'au moins 18 N/25 mm.

10. Matériau composite selon l'une quelconque des revendications précédentes, **caractérisé en ce que** la couche de film (2) respire mais est étanche aux liquides, de sorte que le matériau composite respire également, mais est étanche aux liquides.

11. Matériau composite selon la revendication 10, **caractérisé en ce que** la feuille (2) laisse passer la vapeur d'eau par le processus de chimisorption.

12. Matériau composite selon la revendication 10, **caractérisé en ce que** la feuille (2) présente des micropores pour laisser passer la vapeur d'eau.

13. Matériau composite selon la revendication 12, **caractérisé en ce que** les micropores présentent un diamètre de 0,2 à 10 µm.

14. Matériau composite selon l'une quelconque des revendications précédentes, **caractérisé en ce qu'**au moins la couche de film (2) présente des macropores au moins par segments.

15. Matériau composite selon la revendication 14, **caractérisé en ce que** le composite fibres textiles/microfibres présente également des macropores, de telle sorte que des macropores du composite fibres textiles/microfibres et des macropores de la couche de film (2) se forment par des ouvertures s'étendant à travers le matériau composite.

16. Utilisation d'un matériau composite selon l'une ou plusieurs des revendications précédentes comme couche retenant les liquides dans un article d'hygiène à usage unique.

17. Utilisation selon la revendication 16, **caractérisée en ce que** l'article d'hygiène est une couche, une culotte de propreté, une garniture féminine, un protège-slip ou une protection contre l'incontinence.

18. Utilisation selon la revendication 16 ou 17, **caractérisée en ce que** le matériau composite est utilisé comme feuille dorsale.

19. Utilisation selon la revendication 18, **caractérisée en ce que** la couche de microfibres (6) est disposée sur la face extérieure de la feuille dorsale.

20. Procédé pour fabriquer le matériau composite selon l'une quelconque des revendications 1 à 15, **caractérisé par** les étapes du procédé suivantes :
- Formation d'une couche de fibres textiles (4) avec une structure superficielle ouverte
- Pose de microfibres (6) sur la couche de fibres textiles (4)
- Consolidation de la couche de fibres textiles/microfibres formée par action de pression et d'une température qui est située au-dessus du point de ramollissement des microfibres et/ou des fibres textiles
- Pose d'une feuille préfabriquée (2) côté fibres textiles sur le composite fibres textiles/microfibres préfabriqué
- Consolidation du composite fibres textiles/microfibres avec la feuille par action de pression et d'une température qui se situe au-dessus du point de ramollissement au moins de la feuille.

21. Procédé pour fabriquer le matériau composite selon l'une quelconque des revendications 1 à 15, **caractérisé par** les étapes de procédé suivantes :
- Formation d'une couche de fibres textiles (2) avec une structure superficielle ouverte
- Pose de microfibres (6) sur la couche de fibres textiles
- Consolidation de la couche de fibres textiles/microfibres par action de pression et d'une température qui est située au-dessus du point de ramollissement des microfibres et/ou des fibres textiles,
- Extrusion directe d'une feuille (2) côté fibres textiles sur le composite fibres textiles/microfibres ainsi formé
- Consolidation du composite fibres textiles/microfibres.

## Claims

1. Composite material produced as a fluid-retaining layer in a sanitary article or medical product, with a first layer of substantially endless spun fibres with a diameter of 15-35 µm and with a second film layer and with a third laminar-wise layer (6) for forming a three-layer composite material on the side of the spun fibre layer (4) turned away from the film layer (2) made from microfibres with a diameter of less than 10 µm, wherein the microfibres of this third microfibre layer (6) grip into the surface structure of the spun fibre layer (4) in such a manner that the mean gap (D') between microfibre layer (6) and film layer (2) is less than the thickness Dₛₚ of the sandwich-like enclosed spun fibre layer (4), while the microfibres are applied directly in the meltblown process onto the surface structure of the spun fibre layer (4).

2. Composite material according to claim 1, **characterized in that** the restraining or adhesion force of a Velcro material in relation to the outer side of the composite material formed by the microfibre layer (6) is less than 20 cN/25mm, preferably less than 10 cN/25mm, in particular preferably less than 5 cN/25mm.

3. Composite material according to claim 1 or 2, **characterized in that** the film layer (2) also grips into the three-dimensional surface structure of the spun fibre layer (4).

4. Composite material according to any one of the aforementioned claims, **characterized in that** the basis weight of the composite material amounts to 20-45 gm/m², preferably 25-40 gm/m².

5. Composite material according to any one of the aforementioned claims, **characterized in that** the basis weight of the composite material amounts to 30-35 gm/m².

6. Composite material according to any one of the aforementioned claims, **characterized in that** the basis weight of the microfibre layer (6) amounts to 3-10 gm/m², preferably 4-6 gm/m².

7. Composite material according to any one of the aforementioned claims, **characterized in that** the basis weight of the spun fibre layer (4) amounts to 15-25 gm/m², preferably 18-22 gm/m².

8. Composite material according to any one of the aforementioned claims, **characterized in that** the thickness of the film layer (2) amounts to 9-20 µm, preferably 12-17 µm.

9. Composite material according to any one of the aforementioned claims, **characterized in that** the ultimate tensile strength of the composite material amounts to at least 15 N/25mm, preferably at least 18 N/25mm.

10. Composite material according to any one of the aforementioned claims, **characterized in that** the film layer (2) is breathable but liquid-tight, so that the composite material is also breathable but liquid-tight.

11. Composite material according to claim 10, **characterized in that** the film (2) lets through water vapour by the process of chemical absorption.

12. Composite material according to claim 10, **characterized in that** the film (2) exhibits micro-pores for letting through water vapour.

13. Composite material according to claim 12, **characterized in that** the micro-pores exhibit a diameter of 0.2-10 µm.

14. Composite material according to any one of the aforementioned claims, **characterized in that** at least the film layer (2) exhibits macro-pores at least in sections.

15. Composite material according to claim 14, **characterized in that** the spun fibre/micro fibre composite components also exhibit macro-pores, in such a manner that macro-pores of the spun fibre/microfibre composite components and macro-pores of the film layer (2) form openings extending through the composite material.

16. Use of a composite material according to one or more of the above claims as a fluid-retaining layer for a disposable sanitary article.

17. Use according to claim 16, **characterized in that** the sanitary article is a nappy, training pant, sanitary towel, panty-liner or incontinent care pad.

18. Use according to claim 16 or 17, **characterized in that** the composite material is employed as a backing sheet.

19. Use according to claim 18, **characterized in that** the microfibre layer (6) is arranged on the outer side of the backing sheet.

20. Method for producing the composite material according to any one of claims 1-15 **characterized by** the following process steps:
- Formation of a spun fibre layer (4) with an open surface structure
- Application of microfibres (6) onto the spun fibre layer (4)
- Solidification of the microfibre/spun fibre layer formed by the action of pressure and temperature, which is above the softening point of the microfibres and/or the spun fibres
- Application of a fabricated film (2) on the spun fibre-side onto the microfibre /spun fibre composite fabricated in such a way
- Solidification of the microfibre/spun fibre composite with the film by the action of pressure and temperature, which is above the softening point at least of the film.

21. Method for producing the composite material according to any one of claims 1-15 **characterized by** the following process steps:
- Formation of a spun fibre layer (2) with an open surface structure
- Application of microfibres (6) onto the spun fibre layer
- Solidification of the microfibre/spun fibre layer formed by the action of pressure and temperature, which is above the softening point of the microfibres and/or the spun fibres
- Direct extrusion of a film (2) on the spun fibre side onto the microfibre/spun fibre composite formed in such a way
- Solidification of the microfibre/spun fibre film composite
